Europäisches Patentamt

European Patent Office

Office européen des brevets

(11) Veröffentlichungsnummer : **0 415 298 B1**

(12) **EUROPÄISCHE PATENTSCHRIFT**

(45) Veröffentlichungstag der Patentschrift :
**08.11.95 Patentblatt 95/45**

(21) Anmeldenummer : **90116325.3**

(22) Anmeldetag : **25.08.90**

(51) Int. Cl.⁶ : **G01N 33/92, C12Q 1/60, G01N 33/52**

(54) **Verfahren zur Bestimmung von HDL-Cholesterin mittels eines Schnelldiagnostikums mit integriertem Fraktionierschritt.**

(30) Priorität : **01.09.89 DE 3929032**

(43) Veröffentlichungstag der Anmeldung :
**06.03.91 Patentblatt 91/10**

(45) Bekanntmachung des Hinweises auf die
Patenterteilung :
**08.11.95 Patentblatt 95/45**

(84) Benannte Vertragsstaaten :
**AT BE CH DE DK ES FR GB GR IT LI LU NL SE**

(56) Entgegenhaltungen :
**EP-A- 0 045 476**
**EP-A- 0 319 250**
**DE-A- 3 130 749**
**DERWENT PUBLICATIONS Ltd., London (GB);**
**Database WPIL, Woche 8809; Acc. Nr.**
**88-061491/**
**METHODS IN ENZYMOLOGY, Teil B, Band 129,**
**1986, New York, NY (US); BACHORIK et al.,**
**Seiten 78-100/**
**DERWENT PUBLICATIONS Ltd., London (GB);**
**Database WPIL, Woche 9039; Acc Nr.**
**90-295783/**
(73) Patentinhaber : **BOEHRINGER MANNHEIM GMBH**
**D-68298 Mannheim (DE)**

(72) Erfinder : **Rittersdorf, Walter, Dr.**
**Kasseler Strasse 6**
**D-6800 Mannheim 31 (DE)**
Erfinder : **Deneke, Ulfert, Dr.**
**Birkenweg 5**
**D-6149 Rimbach-Zotzenbach (DE)**
Erfinder : **Hiller, Gerhard, Dr.**
**Glücksburger Weg 236**
**D-6800 Mannheim 31 (DE)**
Erfinder : **Merdes, Hartmut, Dr.**
**Schusterstrasse 5A**
**D-6900 Heidelberg 1 (DE)**
Erfinder : **Bücker Klaus**
**Königsbergerstrasse 4**
**D-6806 Viernheim (DE)**
Erfinder : **Göbbert, Uwe, Dr.**
**Fröhlichstrasse 27a**
**D-6800 Mannheim 31 (DE)**

## Beschreibung

Die Erfindung betrifft ein Verfahren zur quantitativen Bestimmung von HDL (High Density Lipoprotein) in biologischen Flüssigkeiten und ein dafür geeignetes Mittel.

Einer der bekannten Parameter für die Risikoabschätzung bezüglich der Ausprägung einer koronaren Herzerkrankung ist das Gesamtcholesterin im Blut, Plasma oder Serum. Jedoch ist die Konzentration des Gesamtcholesterins für die individuelle Risikoabschätzung nur bedingt geeignet. Aussagekräftiger ist die Quantifizierung des Cholesterins in den Lipoproteinen niedriger Dichte (Low Density Lipoproteins = LDL) einerseits und den Lipoproteinen hoher Dichte (High Density Lipoproteins = HDL) andererseits. Epidemiologische und klinische Studien haben nämlich ergeben, daß zwischen LDL-Cholesterin und koronarer Herzerkrankung eine positive, zwischen HDL-Cholesterin und koronarer Herzerkrankung dagegen eine negative Korrelation besteht.

In erster Näherung ist eine Bestimmung sowohl des HDL- als auch des Gesamtcholesterins für eine Risikoabschätzung ausreichend. In der diagnostischen Praxis wird zur Zeit vorzugsweise dieser Weg beschritten.

Zur separaten Bestimmung von HDL-Cholesterin müssen die anderen vorhandenen Lipoproteinklassen (LDL, VLDL, Chylomikronen) abgetrennt werden. Mögliche Trennmethoden basieren auf Unterschieden in den Schwebedichten (sequentielles Flotieren oder Gleichgewichtssedimentation, beides in der Ultrazentrifuge), auf unterschiedlichen Oberflächenladungen (Elektrophoresen auf Papier oder Agarose als Träger) oder auf Unterschieden in den Apolipoproteinen (immunchemische Methoden unter Verwendung spezifischer Antikörper). Alle diese Trennmethoden sind teuer, aufwendig und in Routinelabors nicht etabliert. Billig, relativ einfach handzuhaben und daher weitverbreitet sind Fällungsreaktionen (in Monographs on Atherosclerosis, Vol. 11 (1982), Clackson, T.B., Kritchevsky, D., Pollak, O.J. eds; Lipoprotein Precipitation, Burstein, M., Legmann, P. und in Meth. in Enzymology, Vol. 129 (1986)), deren Spezifität von der Partikeldimension und der Oberflächenladung abhängt. Als Fällungsreagenzien dienen polymere Substanzen, die in der Regel polyanionischen Charakter haben, aber es sind auch ladungsfreie Polymere geeignet. Die Polyanionen benötigen zur Entfaltung ihrer präzipitierenden Wirkung normalerweise zweiwertige Kationen, die ladungsfreien nicht.

Die gewählten Versuchsanordnungen und die eingesetzten Konzentrationen der kombinierten Fällungsmittel haben das Ziel, alle Lipoproteine außer HDL quantitativ in Präzipitate zu überführen, diese Präzipitate in geeigneter Form von Flüssigteil der Probe abzutrennen und danach im Flüssigteil der Probe das HDL über eine Cholesterinmessung zu quantifizieren. Dazu wird, je nach Ausführungsform des Testes, ein festgelegtes Volumen Fällungsmittel (geeigneter Konzentrationen) mit einem festgelegten Volumen der zu bestimmenden Probe intensiv gemischt. Stand der Technik ist es, allein der Präziptiation wenigstens 10 Minuten Reaktionszeit einzuräumen und erst nach Ablauf dieser Zeitspanne, durch Zentrifugation eine Trennung sedimentierter Nicht-HDL-Lipoproteinpräzipitate und im Flüssigteil bleibender HDL herbeizuführen. Auch der Zentrifugationsschritt benötigt einige Zeit.

Dieses Verfahren ist für eine routinemäßige Untersuchung viel zu zeitaufwendig. Außerdem erfordern Zentrifugationsschritte mit nachfolgender Abtrennung des Überstandes aufwendige Zusatzgeräte und einen Umfüllschritt. Der erforderliche Pipettiervorgang, mit dem eine genau bestimmte Menge des Überstandes entnommen wird, ist darüber hinaus eine Fehlerquelle, die zu weniger genauen Meßergebnissen führen kann.

Es war daher Aufgabe der Erfindung, die Nachteile des Standes der Technik zu vermeiden und ein Verfahren zur Abtrennung von Nicht-HDL-Lipoproteinen aus biologischen Flüssigkeiten zur Verfügung zu stellen, das schneller und ohne aufwendige Zusatzgeräte durchführbar ist und das eine schnellere und einfachere HDL-Cholesterin-Bestimmung möglich macht.

Die Aufgabe wird gelöst durch ein Verfahren zur Abtrennung von Nicht-HDL-Lipoproteinen aus biologischen Flüssigkeiten, wobei die Nicht-HDL-Lipoprotein enthaltende biologische Flüssigkeit auf einen durchströmungsfähigen Träger aufgebracht wird, der ein Fällungsmittel für Nicht-HDL-Lipoproteine ablösbar enthält. Ebenfalls Gegenstand der Erfindung ist ein Mittel zur Abtrennung von Nicht-HDL-Lipoproteinen und ein Schnelldiagnostikum, das dieses Mittel enthält.

Es wurde gefunden, daß die Präzipitation der Nicht-HDL-Lipoproteine mit gängigen, bekannten Fällungsmitteln besonders schnell und spezifisch verläuft, wenn diese Fällungsmittel auf einen durchströmungsfähigen Träger in fein verteilter Form ablösbar aufgebracht werden und die Probe diesen Träger durchströmen muß. Im allgemeinen ist die Präzipitation in weniger als 1 Min. abgeschlossen. Als durchströmungsfähige Träger sind anzusehen: Papiere, Gewebe aus synthetischen Fasern, wie z.B. Polyester oder Polyamid oder andere, Gewebe aus natürlichen Fasern wie z.B. Baumwolle, Seide oder andere, oder Gemische dieser Materialien. Dabei können die Gewebe monofil oder multifil aufgebaut sein, bevorzugt werden multifile Ausführungen.

Bevorzugt weisen die Fasern, die den durchströmungsfähigen Träger aufbauen, Faserdurchmesser von 3 bis 100, bevorzugt 5 bis 50 µm auf. Der Träger hat insbesondere ein Flächengewicht von 10 bis 100, bevorzugt 10 bis 50 g/m² bei einer Dicke von 0.030 bis 0.150 mm, und das Wasseraufnahmevermögen liegt bei 25 bis 100, bevorzugt 40 bis 70 g/m² bei oben genannter Dicke.

Weitere geeignete Träger sind durchströmungsfähige Anordnungen von Glasfasern und Gemischen von Glasfasern mit oben genannten Fasern - vorzugsweise jedoch in Vliesform gebracht - und Membranen unterschiedlicher Ausführungen. Die Membranen müssen hydrophile Eigenschaften, eine Dicke zwischen 20 - 250 µm, bevorzugt 70 - 150 µm, und Porenweiten zwischen 0,2 - 20 µm, bevorzugt 5 - 15 µm, besitzen.

Der Flüssigkeitstransport in diesen Trägern beruht auf Kapillarkräften.

Die Fällungsmittel werden auf den Träger bevorzugt durch Tränken des Trägers mit einer Lösung, Emulsion oder Suspension des Fällungsmittels und anschließendem Trocknen aufgebracht. Dabei können auch noch andere nützliche Hilfsstoffe, wie pH-Puffersubstanzen oder Netzmittel etc. aufgebracht werden.

In dieser Anordnung sind als Fällungsmittel alle die chemischen Verbindungen geeignet, die auch bei "naßchemischer" Ausführung zur Lipoproteinpräzipitation benützt werden, solange sie sich in der Probenflüssigkeit schnell genug lösen. Bekannt sind insbesondere bestimmte Polyanionen in Kombination mit zweiwertigen Kationen. Dazu gehören beispielsweise die Kombinationen von Phosphowolframsäure und Magnesiumchlorid, von Heparin und Mangan (II)-chlorid, oder von Dextransulfat und Magensiumchlorid. Es ist zu bemerken, daß prinzipiell jedes der Polyanionen mit jedem der drei Kationen ($Mg^{2+}$, oder $Mn^{2+}$, oder $Ca^{2+}$) kombinierbar ist, daraus jedoch etwas unterschiedliche Fällungskapazitäten gegenüber Lipoproteinen resultieren (Burstein, 1986). Zur gezielten Ausfällung der Nicht-HDL-Lipoproteine kann die Konzentration des gewählten Kations entsprechend angepaßt werden. Auch die molekulare Größe des eingesetzten Polyanions beeinflußt die Fällungskapazität gegenüber Lipoproteinen und muß bei der Wahl der Konzentration berücksichtigt werden. Zum Beispiel ist der Einsatz von Dextransulfat mit dem Molekulargewicht 500 000 und 50 000 bekannt. Beide sind auch zur Applikation auf durchströmungsfähigen Trägern geeignet, bevorzugt ist jedoch Dextransulfat mit einem Molekulargewicht von 50 000 in der Kombination mit $Mg^{2+}$, wobei $Mg^{2+}$ wiederum vorzugsweise in der Form von Magnesiumacetat eingebracht wird. Jedoch sind prinzipiell auch Magnesiumsulfat, Magensiumchlorid und Magnesiumaspartat zu verwenden.

Die Konzentration der Fällungsmittel kann genau auf das zu untersuchende Probenvolumen abgestimmt werden.

Zur Ausfällung der Nicht-HDL-Lipoproteine wird eine Probe mit dem durchströmungsfähigen Träger, der die Fällungsmittel enthält, in Kontakt gebracht. Die Fällungsreaktion wird dadurch gestartet. Die Zeitspanne, während der die Probe im Fällungsmittelträger mit dem Fällungsmittel in Kontakt ist, läßt sich in einfacher Weise durch die Variation der Saugkraft über die Zwischenraumvolumina bzw. Hydrophilie des Trägers einstellen. Den erzielten Fließgeschwindigkeiten der Probe durch den Träger kommt bezüglich der Leistungsfähigkeit des Tests eine große Bedeutung zu. Nach weniger als einer Minute, in günstigen Fällen schon nach ca. 10 sec., ist die Präzipitatbildung vollendet und kann die Flüssigkeit aus dem Trägermaterial entfernt werden. Dies kann kontinuierlich oder diskontinuierlich beispielsweise durch Absaugen geschehen. Bevorzugt wird die Flüssigkeit durch Kapillarkräfte in einen weiteren Träger gesaugt, in dem die Abtrennung der ausgefällten Nicht-HDL-Lipoproteine stattfindet. Dazu ist erforderlich, daß die Träger miteinander in Kontakt stehen.

Es wurde gefunden, daß Lipoproteinpräzipate, die durch die Einwirkung einer Kombination von Polyanion und genanntem zweiwertigen Kation auf biologische Proben zustand kommen, in einem Flechtwerk aus Fasern zurückgehalten werden können.

Die Anordnung der Fasern im Flechtwerk ist bevorzugt ungeordnet. Als Fasern kommen bevorzugt Glasfasern, Cellulosefasern, Polyamidfasern und Polyesterfasern oder Gemische davon in Frage. Besonders bevorzugt sind Glasfasern und Gemische mit den oben genannten Fasern. Bevorzugt weisen die Fasern, die das Geflecht aufbauen, Durchmesser von 0.2 bis 10,0 besonders bevorzugt von 0,5 bis 5,0 µm auf. Das Geflecht hat ein Flächengewicht von 20 bis 50, bevorzugt 23 bis 30 g/m² und ein Wasseraufnahmevermögen von 250 bis 500 g/m², besonders bevorzugt von 320 bis 420 g/m² bei einer Dicke von 0.19 bis 0.23 mm auf.

Glasfaserenthaltende Systeme, ihre mögliche räumliche Anordnung und die Dimensionen der Fasern sind in DE 30 29 579 im einzelnen beschrieben. Weiterhin kann das Geflecht aus Fasern verfestigt werden, indem man geeignete Bindemittel, entweder anorganischer Natur (z.B. Wasserglas) oder organischer Natur (z.B. Polymere wie Polyvinylacetat, Polyacrylsäureester oder ähnliches) zusetzt. Diese Zusätze bewirken unter anderem das Verkleben von Fasern an den Stellen, an denen sie miteinander im Kontakt sind und verbessern auf diesem Wege die mechanische Stabilität des Fasergeflechts.

Der Flüssigteil der applizierten Probe breitet sich ungestört im ganzen Geflecht aus und kann bei geeigneter geometrischer Anordnung, beziehungsweise bei Überwindung der dem Geflecht innewohnenden Kapillarkräfte, dieses auch verlassen. Auf diesem Wege kann also mit einem einfachen Hilfsmittel die Trennung zwischen präzipitierten Nicht-HDL-Lipoproteinen und nichtpräzipitiertem HDL bewerkstelligt werden. Biologische Flüssigkeiten, aus denen Nicht-HDL-Lipoproteine auf erfindungsgemäße Weise abgetrennt werden können, sind insbesondere Vollblut, Serum und Plasma.

Das erfindungsgemäße Verfahren zur Abtrennung von Nicht-HDL-Lipoproteinen kann besonders vorteilhaft in Verfahren zur quantitativen Bestimmung von HDL-Cholesterin auf Teststreifen eingesetzt werden. Dazu wird die Flüssigkeit, aus der in oben beschriebener Weise die Nicht-HDL-Lipoproteine abgetrennt wurden, in Kontakt gebracht mit Reagenzien, die für die Durchführung der Bestimmungsreaktion, beispielsweise einer Nachweisreaktion für Cholesterin, erforderlich oder/und nützlich sind. Solche Reagenzien sind dem Fachmann bekannt, beispielsweise aus der EP-B-0016387, und können der zu erwartenden Cholesterinkonzentration (0-100 mg/dl) angepaßt werden. Sie können beispielsweise auch in Form eines Films oder eines Überzugs auf einem porösen Träger vorliegen. Solche Ausführungsformen der Reagenzdarreichung sind dem Fachmann bekannt.

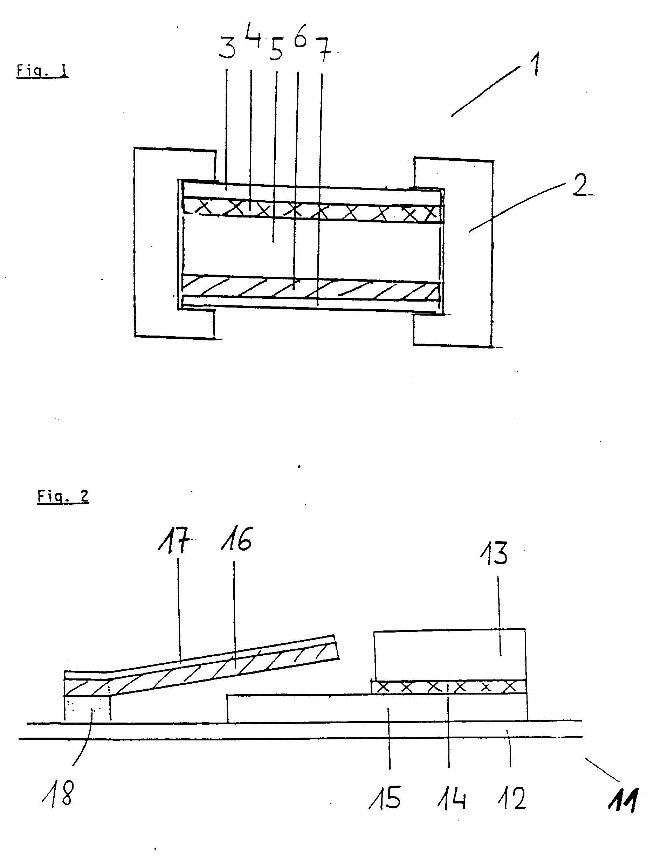

Eine Ausführungsform eines Verfahrens zur HDL-Cholesterin-Bestimmung, welches die bevorzugte Ausführungsform der Abtrennung der Nicht-HDL-Lipoproteine enthält, ist in Fig. 1 gezeigt. Ein Schnelldiagnostikum (1) enthält in einem Gehäuse (2) mehrere Trägerschichten.

Unter einem mit Fällungsmitteln imprägnierten durchströmungsfähigen Träger (4) liegt ein Fasergeflecht (5) mit der dem gewählten Probenvolumen angepaßten Trennkapazität für Lipoproteinpräzipitate. Daran schließt ein geeigneter saugfähiger Cholesterinnachweisfilm (6) an, der imstande ist, präzipitatfreie "Flüssigkeit" aus dem Fasergeflecht aufzunehmen. Der Nachweisfilm ist auf eine transparente Folie (7) beschichtet. Gestartet wird die Bestimmung mit der Applikation der Probenflüssigkeit über dem Fällungsmittelträger. Von der Folienseite des Aufbaus aus kann ein sich entwickelndes Meßsignal visuell oder photometrisch ausgewertet werden.

Diese einfachste Ausführung ist für die Untersuchung von Vollblut wenig geeignet. Soll Vollblut als Probenmaterial Verwendung finden, so werden bevorzugt in einer dem Fällungsmittelträger vorgeschalteten Schicht (3) die zellulären Blutbestandteile abgetrennt, z.B. durch Mittel, die in DE-A-30 29 579 näher beschrieben sind.

Im Hinblick auf die bekannte Temperaturabhängigkeit enzymatischer Reaktionen kann die geometrische Anordnung der Testausführung auch so gewählt werden, daß der Cholesterinnachweis von den davorliegenden Reaktionsschritten zeitlich getrennt werden kann. Dies erlaubt auch eine kontrollierte Temperierung des Nachweisschrittes. Ein entsprechender Testaufbau ist in seinen Grundzügen in DE-A-3130749 beschrieben. Die bevorzugte Ausführung eines HDL-Schnelldiagnostikums in Form eines Teststreifens (11) ist in Fig. 2 dargestellt. Auf einer Grundfolie (12) sind ein Fasergeflecht (15), ein durchströmungsfähiger Träger mit Fällungsmittel (14) sowie eine Trennschicht für zelluläre Bestandteile (13) übereinander angebracht. Das Fasergeflecht (15) ragt etwas in Richtung auf eine Klappe unter den Trägern (13) und (14) hervor. Die Klappe, bestehend aus einer transparenten Folie (17) und einem Cholesterinnachweisfilm (16) ist über eine Klebestelle (18) an der Grundfolie (12) befestigt.

Die zu untersuchende Probe wird auf die Schicht (13) aufgegeben und fließt unter Abtrennung der Nicht-HDL-Lipoproteine durch die Schichten (14) und (15) in das Geflecht (15) unter der Klappe. Die Nachweisreaktion wird durch Aufdrücken der Klappe mit der Nachweisschicht (16) auf die Schicht (15) gestartet. Die Veränderung der Farbe, kann mittels eines Photometers, auch eines Reflexionsphotometers, verfolgt werden.

Im Unterschied zu der in Fig. 1 skizzierten Ausführungsform kommt hier auch eine laterale Trennkapazität des verwendeten Fasergeflechts zur Wirkung.

Das erfindungsgemäße Verfahren hat gegenüber den bekannten Verfahren weitere große Vorteile. Es ist möglich, auch besonders kleine Flüssigkeitsvolumina einzusetzen. Die Handhabung des erfindungsgemäßen Mittels ist sehr einfach. Es sind nur zwei grundlegende Handlungen erforderlich, nämlich die Aufgabe einer flüssigen Probe und die Ablesung eines Meßwerts nach einer bestimmten Zeit. Alle Geräte außer einem geeigneten Photometer im Falle quantitativer Auswertung können entfallen. Umfüllschritte entfallen. Alle Sorten von Blut sind einfach einsetzbar, auch Vollblut, wenn ein Abtrennvlies für Zellen vorgeschaltet wird. Sollten der zu untersuchenden Probe Antikoagulantien zugesetzt worden sein, ist ein Ausgleich der dadurch hervorgerufenen Einflüsse auf die Bestimmung empfehlenswert. Die Abtrennung von Nicht-HDL-Lipoproteinen kann in weniger als 60 sec erfolgen. Die Dosierung des Probevolumes wird stark vereinfacht. Im folgenden werden Beispiele für die Erfindung gegeben.

Beispiel 1

Fällungsmittelträger (14 bzw. 4) für EDTA-Plasma

Ein multifiles Polyestergewebe (100 /um Maschenöffnung, 105 $\mu$m Gewebedicke, 55 Fäden pro cm, mit 815 1 Wasserdurchlaß pro m$^2$ und sec) wird mit einer Lösung folgender Zusammensetzung imprägniert:

| | |
|---|---|
| Hepes-Puffer, 50 mM; pH 7,0 | 78,00 g |
| Magnesiumacetat x 4H$_2$O | 15,68 g |
| Dextransulfat (MW 50000) | 2,57 g |

Rinderserumalbumin 1,78 g

Es wird ein Auftrag von ca. 57 ml/m$^2$ erzielt.

Nach der Trocknung unter einem Warmluftstrom wird das imprägnierte Gewebe passend zur entsprechenden Testausführung in geeignete Flächeneinheiten geschnitten und in den Testaufbau integriert.

Beispiel 2

Fällungsmittelträger (14 bzw. 4) für Serum

Hier ist beim Vorgehen analog zu Beispiel 1 folgende Tränklösung zu benützen:

Hepes-Puffer, 50 mM; pH 7,0 78,00 g
Magnesiumacetat x 4H$_2$O 10,10 g
Dextransulfat (MW 50000) 2,57 g
Rinderserumalbumin 1,78 g
H$_2$O 5,58 g

Beispiel 3

Fällungsmittelträger (14 bzw. 4) für Blut

Ein Papier geeigneter Dicke und Saugfähigkeit, z.B. Teebeutelpapier mit 12 g/cm$^2$ Flächengewicht und 50 µm Dicke, wird mit folgender Tränklösung imprägniert:

Hepes-Puffer, 50 mM; pH 7,0 70,20 g
Magnesiumacetat x 4H$_2$O 4,78 g
Dextransulfat (MW 50000) 2,54 g
Rinderserumalbumin 1,60 g
H$_2$O 7,80 g

Mit diesem Träger wird ein Auftrag von ca. 63 ml/m$^2$ erzielt.

Beispiel 4

Nachweisfilm

Zur Herstellung eines Reagenzfilmes zur Quantifizierung von HDL-Cholesterin wird eine Dispersion folgender Zusammensetzungen bereitet:

| | |
|---|---|
| K/Na-Phosphatpuffer, 0,5 M; pH 7,0 | 17,14 g |
| Keltrol F | 0,19 g |
| $TiO_2$ (Pulver) | 1,31 g |
| Dioctylnatriumsulfosuccinat | 0,40 g |
| Polyvinylpropionat-Dispersion (50 % in $H_2O$) | 11,70 g |
| Diatomenerde (Celatom MW 25) | 17,65 g |
| Phenylsemicarbazid | 0,025 g |
| 2(4-Hydroxy-3,5-dimethoxyphenyl)-4-(4-dimethyl-aminophenyl)-5-methyl-imidazol-dihydrochlorid | 0,061 g |
| Methanol | 1,74 g |
| $H_2O$ | 46,28 g |
| Cholesterinesterase | 23700 U |
| Cholesterinoxidase | 6500 U |
| Peroxidase | 230000 U |
| Hexanol | 2,07 g |

Die Dispersion wird als 300 µm dicke Schicht auf eine Polycarbonat-Folie aufgetragen und mit Warmluft getrocknet. Die resultierende Reagenzschicht ergibt mit cholesterinhaltigen Flüssigkeiten in Abhängigkeit von ihrem Cholesteringehalt abgestufte blaue Färbungen.

| Cholesterinkonzentration | Remission, R% (bei Messungen im Reflexionsphotometer Reflotron R) |
|---|---|
| 0 | 70,0 |
| 20 | 43,0 |
| 40 | 29,0 |
| 60 | 22,0 |
| 80 | 18,5 |
| 100 | 16,0 |

Beispiel 5

Fasergeflecht

Gemisch aus Borsilikat-Glasfasern vom Faserdurchmesser ca. 0.6 µm und Cellulosefasern vom Faserdurchmesser ca. 4 µm, vorzugsweise im Verhältnis 9 zu 1. Flächengewicht ca. 25 g/m² bei einer Geflechtsdicke von ca. 0.21 mm; Wasseraufnahmevermögen der Geflechte ca. 370 g/m².

Beispiel 6

Herstellung eines Schnelldiagnostikums (11) zur Bestimmung von HDL

Wird die in Fig. 2 skizzierte Testausführung gewählt, lassen sich für die einzelnen Schichten folgende Maße angeben:

12        : 100 x 6 mm

13        : 5 x 6 mm (Borsilikatvlies gemäß DE-A-3029579, Flächengewicht ca. 60 g/m$^2$)

14        : 6 x 6 mm

15        : 16 x 6 mm

16/17   : 15 x 6 mm

Diese Ausführungsform ist für ein Probenvolumen zwischen 28 - 32 μl geeignet und besonders vorteilhaft.

**Patentansprüche**

1.     Verfahren zur Abtrennung von nicht-HDL-Lipoproteinen aus biologischen Flüssigkeiten, dadurch gekennzeichnet, daß die nicht-HDL-Lipoproteine enthaltende biologische Flüssigkeit auf einen durchströmungsfähigen Träger aufgebracht wird, der mit einem in der biologischen Flüssigkeit löslichen Fällungsmittel für nicht-HDL-Lipoproteine imprägniert ist und die biologische Flüssigkeit nach Durchführung des Fällungsschrittes mit einem weiteren durchströmungsfähigen Träger in Kontakt gebracht wird.

2.     Verfahren zur Bestimmung von HDL-Cholesterin in einer nicht-HDL-Lipoprotein enthaltenden Probe, dadurch gekennzeichnet, daß die Probe auf einen durchströmungsfähigen Träger aufgebracht wird, der mit einem in der Probe löslichen Fällungsmittel für nicht-HDL-Lipoproteine imprägniert ist, die daraus austretende Flüssigkeit anschließend mit einem weiteren durchströmungsfähigen Träger in Kontakt gebracht wird, der in der Lage ist, ausgefällte Bestandteile zurückzuhalten und die daraus austretende Flüssigkeit auf ihren Gehalt an HDL-Cholesterin untersucht wird.

3.     Mittel zur Abtrennung von nicht-HDL-Lipoproteinen aus biologischen Flüssigkeiten, dadurch gekennzeichnet, daß es einen durchströmungsfähigen Träger enthält, auf dem ein Fällungsmittel für nicht-HDL-Lipoproteine durch biologische Flüssigkeiten ablösbar imprägniert ist, und daß es einen durchströmungsfähigen Träger enthält, der in der Lage ist, ausgefällte Bestandteile zurückzuhalten.

4.     Schnelldiagnostikum zur Bestimmung von HDL-Cholesterin in biologischen Flüssigkeiten enthaltend einen durchströmungsfähigen Träger, der mit einem durch biologische Flüssigkeiten ablösbaren Fällungsmittel für nicht-HDL-Lipoproteine imprägniert ist, einen durchströmungsfähigen Träger, der in der Lage ist, ausgefällte Bestandteile zurückzuhalten und die für die Durchführung der Bestimmungsreaktion erforderlichen Reagenzien.

**Claims**

1.     Process for the separation of non-HDL-lipoproteins from biological liquids, characterised in that the biological liquid containing non-HDL-lipoproteins is applied to a carrier capable of flowthrough which is impregnated with a precipitation agent for non-HDL-lipoproteins soluble in the biological liquid and the biological liquid, after carrying out of the precipitation step, is brought into contact with a further carrier capable of flowthrough.

2.     Process for the determination of HDL-cholesterol in a sample containing non-HDL-lipoproteins, characterised in that the sample is applied to a carrier capable of flowthrough which is impregnated with a precipitation agent for non-HDL-lipoproteins soluble in the sample, the liquid emerging therefrom is subsequently brought into contact with a further carrier capable of flowthrough which is able to retain precipitated components and the liquid emerging therefrom is investigated for its content of HDL-cholesterol.

3.     Agent for the separation of non-HDL-lipoproteins from biological liquids, characterised in that it contains a carrier capable of flowthrough on which is impregnated a precipitation agent for non-HDL-lipoproteins dissolvable by biological liquids and that it contains a carrier capable of flowthrough which is able to retain precipitated components.

7

4. Rapid diagnostic for the determination of HDL-cholesterol in biological liquids containing a carrier capable of flowthrough which is impregnated with a precipitation agent for non-HDL-lipoproteins dissolvable by biological liquids, a carrier capable of flowthrough which is able to retain precipitated components and the reagents necessary for the carrying out of the determination reaction.

## Revendications

1. Procédé de détermination de lipoprotéines non HDL dans des liquides biologiques, caractérisé en ce que l'on applique le liquide biologique contenant les lipoprotéines non HDL sur un support que le liquide peut traverser, qui est imprégné d'un agent de précipitation des lipoprotéines non HDL, soluble dans le liquide biologique, et après avoir réalisé l'étape de précipitation, on met en contact le liquide biologique avec un autre support qu'il peut traverser.

2. Procédé de détermination de cholestérol HDL dans un échantillon contenant des lipoprotéines non HDL, caractérisé en ce que l'on applique l'échantillon contenant les lipoprotéines non HDL sur un support que celui-ci peut traverser, qui est imprégné d'un agent de précipitation des lipoprotéines non HDL, soluble dans l'échantillon, on met en contact ensuite le liquide ainsi obtenu avec un autre support qu'il peut traverser, ce support étant apte à retenir les constituants précipités, et on détermine la teneur en cholestérol HDL du liquide ainsi obtenu.

3. Moyen pour la séparation des lipoprotéines non HDL à partir de liquides biologiques, caractérisé en ce qu'il contient un support que les liquides peuvent traverser, qui est imprégné d'un agent de précipitation des lipoprotéines non HDL, soluble dans les liquides biologiques, et qu'il contient un support que les liquides peuvent traverser, ce support étant apte à retenir les constituants précipités.

4. Agent de diagnostic rapide pour la détermination du cholestérol HDL dans des liquides biologiques, contenant un support que les liquides peuvent traverser, qui est imprégné d'un agent de précipitation des lipoprotéines non HDL, soluble dans les liquides biologiques, un support que les liquides peuvent traverser, qui est apte à retenir les constituants précipités, et les réactifs nécessaires à la réalisation de la réaction de détermination.

Fig. 1

3 4 5 6 7

1

2

Fig. 2

17 16

13

18

15 14 12

11